(19) 

**Europäisches Patentamt**
**European Patent Office**
**Office européen des brevets**

(11) **EP 4 600 362 A1**

(12) **EUROPEAN PATENT APPLICATION**

(43) Date of publication:
**13.08.2025 Bulletin 2025/33**

(21) Application number: **24382121.2**

(22) Date of filing: **07.02.2024**

(51) International Patent Classification (IPC):
**C12N 15/63** *(2006.01)*     **C07K 14/47** *(2006.01)*
**C12N 15/70** *(2006.01)*

(52) Cooperative Patent Classification (CPC):
**C12N 15/70; C07K 14/245; C12N 15/52; C12N 15/63**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Instituto Nacional de Técnica Aeroespacial "Esteban Terradas" 28850 Torrejón de Ardoz (ES)**

(72) Inventors:
- **DÍAZ-RULLO AROCO, Jorge 28850 Torrejón de Ardoz (Madrid) (ES)**
- **GONZÁLEZ PASTOR, José Eduardo 28850 Torrejón de Ardoz (Madrid) (ES)**

(74) Representative: **TRBL Intellectual Property Plaza de Castilla 3, 7°A 28046 Madrid (ES)**

Remarks:
The complete document including Reference Table(s) and the Sequence Listing(s) can be downloaded from the EPO website

(54) **REPORTER GENE CONSTRUCT SENSITIVE TO QUEUOSINE LEVELS**

(57)     The present invention relates to a genetic construct sensitive to queuosine (Q)-tRNA levels within a cell. It is based on the regulation mechanism of the histidine (His) operon, which depends on the queuosine modification of tRNA. The construct includes the promoter of the His operon ($P_{his}$) and the His leader gene (*hisL*). In addition to $P_{his}$ and *hisL,* the genetic construct of the invention comprises a gene encoding for a reporter protein, wherein all NAU codons in the gene encoding for the reporter protein have been modified to NAC codons. In this way, the translation of the reporter gene itself is independent on Q-tRNA levels. The construct can be used as a marker for successful detection of Q-tRNA(His) within the cell and it can also be used to quantify queuosine and/or queuosine precursor in a sample.

| Promoter | ATG---**CACCACCATCATCACCATCAT**---TAG | | | | | C-EGFP | Terminator |
|---|---|---|---|---|---|---|---|
| | *hisL* – (4xCAT + 3xCAC) | | A-B | C-D | E-F | | |

**FIG. 1**

## Description

### FIELD OF THE INVENTION

The present invention lies within the technical field of biotechnology. More specifically, the invention relates to a reporter gene construct sensitive to Q-tRNA levels within a cell, which may be used to measure queuosine (Q) or queuosine precursors in a sample.

### BACKGROUND OF THE INVENTION

Translation of genetic information into proteins requires an efficient and accurate decoding of the messenger RNA (mRNA) by ribosomes and transfer RNAs (tRNAs). Among all RNA species, tRNAs undergo the greatest number of chemically diverse modifications. Growing evidence indicates that tRNA modifications are critical for many aspects of tRNA functions such as folding, stability and decoding, representing an additional level of gene regulation. Despite their essential role in translation, the physiological function of several tRNA modifications is not very well understood.

One such modification is queuosine. Queuosine (Q) is a nucleoside that can replace guanosine at position 34 in the anticodon of the tyrosine, histidine, asparagine, and aspartic acid tRNAs to fine tune protein translation. The codons coding for these amino acids are UAU/UAC (Tyr), CAU/CAC (His), AAU/AAC (Asn) and GAU/GAC (Asp), which are collectively represented as NAU/NAC. Q is found in Bacteria and Eukarya, although its de *novo* biosynthesis only occurs in Bacteria. Q biosynthesis pathway starts with five sequential modifications of GTP catalysed by the enzymes FolE, QueD, QueE, QueC and QueF to obtain the $preQ_1$ precursor. This precursor is incorporated to tRNA by the tRNA guanine transglycosylase (TGT) and finally transformed into Q by QueA and QueG/QueH. Although many species can synthesize Q *de novo,* salvage of Q precursors also occurs. Some species use the YhhQ transporter for importing $preQ_0$ and $preQ_1$ precursors. Certain bacteria capture queuine (q), the Q nucleobase, and transform it into $preQ_1$. Other bacteria and eukaryotes directly replace the guanine at position 34 of the tRNAs with q by using a TGT homolog or a eukaryotic TGT (eTGT), respectively. Bacteria that cannot produce Q *de novo* and eukaryotes need to salvage Q precursors through the microbiome and/or nutrient sources.

Given that Q modification is in the wobble anticodon position critical for codon recognition, one suggested purpose of tRNA Q-modification is modulating NAU codon translation rate. However, its physiological role remains elusive. Multiple studies conducted in eukaryotes have revealed the involvement of tRNA Q-modification in very diverse processes, including pupae maturation in *Drosophila melanogaster,* cell aggregation in *Dictyostelium discoideum*, and antioxidant defence system, hypoxia, cancer, and proliferation in mammals. In bacteria, it has been reported that tRNA Q-modification is involve in resistance to several stressors, such as heat shock, low acidic pH, UV-radiation, perchlorate, and arsenic, as well as in the regulation of the virulence of the pathogen *Shigella flexneri* and the nodule cell infection efficiency of *Sinorhizobium meliloti.* The molecular mechanism underlying these widely diverse and spread phenotypes is not well understood. Previous studies suggest that availability of Q would especially affect the translation of genes enriched in NAU codons (known as "Q-genes"), leading to a variation in the expression levels of the proteins they encode depending on the degree of Q-modification of tRNAs. In this respect, this general mechanism of regulation could be responsible for the wide variety of reported Q-related phenotypes, which may vary depending on the roles of the specific NAU codon-enriched genes in each organism. Moreover, as Q modification is fully dependent on diet or on gut microbiome in multicellular organisms, and Q controls biofilm formation and virulence in bacteria, it is thought that higher levels of Q are associated with certain pathologies related to microbial dysbiosis, such as chronic intestinal diseases.

Therefore, there is an obvious interest in the field for detecting and controlling queuosine levels, not only to shed light on the physiological role of tRNA Q-modifications, but also to help treat bacterial infections and other complex pathologies related to the microbiome. In these regards, there is currently no specific technique for the measurement of queuosine or its precursors. Theoretically, it would be possible to make these measurements using the general techniques of chromatography or mass spectrometry. However, there is no standardised protocol that would allow measuring queuosine using these techniques, which in addition are expensive and require specialised personnel due to their high complexity.

### DESCRIPTION OF THE FIGURES

Figure 1 shows a preferred embodiment of the Q-reporter gene construct of the invention, $P_{his}$-*hisL*-Δ*hisG*:: *egfp(NAC).*

Figure 2 shows the nucleotide sequence of a preferred embodiment of the Q-reporter gene construct of the invention,

$P_{his}$-*hisL*-Δ*hisG*::*egfp(NAC)*, together with the position and a brief description of each element within the construct.

Figure 3 shows a scheme of the queuosine (Q) biosynthetic pathway.

Figure 4 shows the linear fittings of preQ$_1$ concentration vs corrected fluorescence (signal to noise) measurements of M63 medium samples (control; represented as a scatter plot with circular markers) and of the biological samples (10 nM preQ, in M63 medium; represented as a scatter plot with squared markers). The fluorescence measured in the presence of a high concentration of extracellular preQ$_1$ (250 nM) is considered as noise, as tRNA would be Q-modified at a maximum degree in these conditions.

Figure 5 shows the preQ$_1$ concentration obtained for a sample containing M63 medium alone, M63 medium plus 10 nM of preQ$_1$, culture supernatant of an *E. coli* DH10B *queF* mutant strain that does not produce preQ$_1$ (*E. coli* DH10B Δ*queF*), and the same supernatant plus 10 nM of preQ$_1$, incubated in M63 at 37 °C and 200 rpm for 16 h.

Figure 6 shows the preQ$_1$ concentration measured in supernatants (SB) of different *E. coli* strains (ST131, DH10B), in *Staphylococcus aureus* and, in sera from foetal bovine, mouse and human, human urine, and in filtered LB medium.

Figure 7 shows a quantitative relationship between preQ$_1$ concentration and corrected fluorescence of *E. coli* DH10B Δ*queF* $P_{his}$-*hisL*-Δ*hisG*::*egfp(NAC)* strain (A) and linearised (B).

Figure 8 shows linearized quantitative relationship between extracellular preQ$_1$ concentration and fluorescence of *E. coli* DH10B Δ*queF* $P_{his}$-*hisL*-Δ*hisG*::*egfp(NAC)* strain incubated with different concentrations of indole.

Figure 9 shows the calculated values of $V_{max(app)}$ and $K_{M(app)}$ when *E. coli* DH1 0B Δ*queF* $P_{his}$-*hisL*-Δ*hisG*::*egfp(NAC)* strain was incubated with different concentrations of indole.

Figure 10 shows linearized quantitative relationship between extracellular preQ$_1$ concentration and fluorescence of *E. coli* DH10B Δ*queF* $P_{his}$-*hisL*-Δ*hisG*::*egfp(NAC)* strain transformed with plasmid 84 or the two genes harboured by plasmid 84 cloned individually.

Figure 11 shows the calculated values of $V_{max(app)}$ and $K_{M(app)}$ when *E. coli* DH10B Δ*queF* $P_{his}$-*hisL*-Δ*hisG*:: *egfp(NAC)* strain was transformed with plasmid 84 or the two genes harboured by plasmid 84 cloned individually.

## DETAILED DESCRIPTION OF THE INVENTION

The present invention provides a novel artificial gene construction sensitive to Q-tRNA levels in a cell, that can be used to measure queuosine and/or queuosine precursors in a sample. The construction comprises EGFP as a reporter gene under the control of the histidine operon. This construct is sensitive to queuosine because of the regulatory mechanisms of the histidine operon. However, all NAU codons in the reporter protein EGFP have been modified to NAC codons such that expression of EGFP itself is independent of queuosine levels. The invention allows to correlate EGFP(NAC) fluorescence levels with the levels of queuosine or a queuosine precursor in a sample, and the technology makes it possible to measure queuosine at very low concentrations in the nanomolar range. This invention represents a cheap, simple and straightforward tool, and does not require specialised technical personnel or complex or expensive machinery. Instead, it can be used anywhere with minimal laboratory equipment.

Thus, in a first aspect, the invention refers to a genetic construct, wherein the genetic construct comprises:

- the promoter of the histidine operon ($P_{his}$);
- the histidine leader gene (*hisL*);
- the His operon regulatory elements between *hisL* and the following gene of the His operon (*hisG*);
- a gene encoding for a reporter protein replacing *hisG*, and wherein all NAU codons in the gene encoding for the reporter protein have been modified to NAC codons;
- a transcriptional terminator.

The construct of the invention is based on the regulation mechanism of the histidine (His) operon, which involves the promoter of the His operon ($P_{his}$) and the His leader gene (*hisL*). The latter is composed of 17 codons, seven of them encoding for His. Four out of these seven His codons are CAU, and the other three are CAC. When the His levels are high, tRNA(His) are charged with His, the translation of the seven His codons is fast, and this causes the formation of a transcription termination fork called E-F. On the other hand, when the His levels are low, tRNA(His) is poorly charged with His, the translation of the seven His codons is slow, and this prevents the formation of the transcription termination fork E-F, allowing for the His operon transcription (see Johnson et al., 1980. Model for regulation of the histidine operon of Salmonella. Proc. Natl. Acad. Sci. USA, 77, 508-512). It is known that the presence of queuosine (Q) in certain tRNAs alters the translation efficiency of some codons. More precisely, it has been demonstrated that Q enhances codon translation of NAU codons so that when Q is absent NAU codons are not well translated. This mechanism regulates the translation levels of genes containing a high percentage of NAU codons. Therefore, as *hisL* contains a high percentage of CAU codons, its translation depends on the levels of Q-tRNA(His). It follows that the genetic construct of the invention is sensitive to queuosine (Q)-tRNA levels within the cell.

In the context of the instant invention, any transcription terminator capable of operating in the host organism where the gene construct is inserted may be used. Non limiting examples are the transcriptional terminator T0 from phage lambda, the transcriptional terminator T7, or the transcriptional region of the rrnB gene from *E. coli.* Thus, in embodiments of the invention, the transcriptional terminator may be selected from the group consisting of the transcriptional terminator T0 from phage lambda, the transcriptional terminator T7, or the transcriptional region of the rrnB gene from *E. coli.* In a particular embodiment, the transcriptional terminator is the transcriptional terminator T0 from phage lambda.

In the context of the invention, the term "gene encoding for a reporter protein", or "reporter gene", is a gene that can be attached to the regulatory sequence of the His operon in the construct of the invention such that, when introduced into a suitable host cell, expression of the said reporter gene can confer the host cell certain characteristics that are easily identified and/or measured. Detection of expression of the reporter gene may be carried out by any suitable method, according to the specific gene. Suitable methods may include enzymatic assays, histochemistry, fluorescence, microscopy, spectrophotometry, bioluminescence and the like. The gene product of the reporter gene may be, but is not limited to, β-galactosidase, chloramphenicol acetyltransferase, green fluorescent protein, red fluorescent protein, luciferase, etc. As indicated above, all NAU codons in the gene encoding for the reporter protein have been modified to NAC codons so that translation of the reporter gene itself is not depending on Q-tRNA levels. In particular embodiments of the invention, the reporter protein is selected from the group consisting of NAC-β-galactosidase, NAC-chloramphenicol acetyltransferase, NAC-luciferase, RFP (NAC), GFP(NAC), EGFP(NAC), or combinations thereof. In a particular embodiment of the invention, the reporter protein is the Enhanced Green Fluoresce Protein (EGFP). The gene encoding for EGFP is modified to change all the NAU codons by NAC codons [EGFP(NAC)], thus translation of EGFP itself does not depend on Q-tRNA levels. However, EGFP(NAC) in the construct of the invention is under the control of the His operon. Therefore, the levels of EGFP fluorescence can be correlated with the levels of Q-tRNA(His).

In a particular embodiment, the genetic construct of the invention has a sequence with an 80% homology to SEQ ID NO:1. In particular embodiments, the genetic construct of the invention has a sequence with an 80%, 85%, 90%, 95%, 98%, 99% or 100% homology to SEQ ID NO:1. In a particular embodiment, the genetic construct of the invention has the sequence of SEQ ID NO: 1.

In another aspect, the invention relates to a host cell, wherein the host cell can only use exogenous queuosine or an exogenous queuosine precursor selected from the group consisting of 7-cyano-7-deazaguanine (preQ$_0$), 7-aminomethyl-7-deazaguanine (preQ$_1$), and queuine (q), and wherein the host cell comprises a genetic construct according to the instant invention. This limitation of the host cell implies that the cell will necessarily produce Q-tRNA using the exogenous queuosine or queuosine precursors. In a particular embodiment, the host cell is a *ΔfolE, ΔqueD, ΔqueE* y *ΔqueC* mutant. In a particular embodiment, the host cell is a *ΔqueA* y *ΔqueG/H* mutant. In a particular embodiment, the host cell is a *ΔqueF* mutant; that is, a host cell wherein the *queF* gene was deleted to prevent the biosynthesis *de novo* of preQ$_1$. This way, all the Q-tRNA is formed from the exogenous preQ$_1$ of a certain sample from which the concentration of preQ$_1$ wants to be measured. The gene *queF* codifies for the protein NADPH-dependent 7-cyano-7-deazaguanine reductase, wherein the protein catalyses the NADPH-dependent reduction of 7-cyano-7-deazaguanine (preQ$_0$) to 7-aminomethyl-7-deazaguanine (preQ$_1$), a late step in the queuosine pathway. Its UniProt entry in respect of organism *Bacillus subtilis* (strain 168) is 031678. In a particular embodiment, the host cell is a cell wherein the genes *queL* (queuine lyase), *queK* (queuosine hydroxylase) and the transporter *yhhQCt* have been cloned. The *yhhQCt* transporter is a homologue of *E. coli yhhQ,* but with the difference that *yhhQCt* can import free queuine and queuosine. QueL and QueK are two enzymes that catalyse the transformation of free queuosine into queuine (QueK), and queuine into preQ$_1$ (QueL). With these mutations, the host cell can use free Q or q to produce Q-tRNA. Suitable cells to be used as a host cell in the context of the invention irrespective of the specific mutation or mutations may include, without limitation, bacterial cells. In particular embodiments, the host cell is a bacterium of a genus selected from the group consisting of *Escherichia, Bacillus, Pseudomonas, Streptococcus, Streptomyces, Staphylococcus* and *Lactobacillus.* In a particular embodiment, the host cell is a bacterium of a species selected from the group consisting of *Escherichia coli,* and *Bacillus subtilis.* In a particular embodiment, the host cell is a *ΔqueF* mutant strain of *Escherichia coli* or a *ΔqueF* mutant strain of *Bacillus subtilis.*

In another aspect, the invention refers to the use of a genetic construct according to the invention, or of a host cell according to the invention, for measuring the degree of queuosine modification of tRNA in a cell, for detecting queuosine and/or a queuosine precursor in a sample, preferably in a biological sample, or for measuring queuosine and/or a queuosine precursor in a sample, preferably in a biological sample.

In the context of the invention, the degree of queuosine modification may be expressed as a relative value with respect to a measurement used as a reference. Once the reference value is established, the degree of queuosine modification can be compared with this reference value, and thus be assigned a level of "increased" or "decreased". For example, an increase in the degree of queuosine modification above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or more compared with the reference value is considered as an "increased" degree of queuosine modification. On the other hand, a decrease in the degree of queuosine modification below the reference value of at least 0.9-fold, 0.8-fold, 0.7-fold, 0.6-fold, 0.5-fold, 0.2-fold, 0.1-fold, 0.05-fold, 0.025-fold, 0.02-fold, 0.01-fold, 0.005-fold or less compared with the reference value is considered as a "decreased" degree of queuosine modification. "Reference value", as used herein in the context of the invention, refers to a laboratory value used as a reference for values/ data obtained by laboratory examination of representative samples, such as a sample from a standard environment or a sample from a subject or subjects. The reference value or reference level can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject or from the environment being tested, but at an earlier point in time or from a noncancerous tissue. The reference value can be based on a large number of samples or based on a pool of samples including or excluding the sample to be tested. In the context of the invention, detecting queuosine and/or a queuosine precursor in a sample necessarily results in a positive or negative outcome (i.e., a yes or no result), wherein a positive result can be determined when the queuosine concentration is above a certain threshold value. In the context of the invention, measuring queuosine and/or a queuosine precursor in a sample results in a quantifiable result, typically expressed as a concentration value.

As used herein, "sample" refers to a limited quantity of a larger amount of a certain object, which maintains the characteristics and is representative of said object, that is taken for analysis, testing or investigation. "Biological sample" means biological material isolated from a subject (i.e., from an animal or from a human subject) or from a biological environment, such as cellular extracts from bacteria, eukaryotes or archaea; cell culture supernatants; water samples (taken from rivers, lakes, ponds, salt flats, etc.), soils (soil, sediments, etc.), food products, biofilms and/or communities of microorganisms associated with biotic or abiotic surfaces. The biological sample may contain any biological material suitable for detecting and or for measuring queuosine and/or a queuosine precursor. The sample can be isolated from any suitable biological tissue or fluid such as, for example, blood, plasma, serum, sputum, bronchoalveolar lavage, urine or cerebrospinal fluid (CSF). In the case of solid tissue samples, it would be necessary to pre-treat the sample to liquefy it, resuspend the sample in an appropriate buffer and treat the sample under a mechanical or sonication procedure.

In another aspect, the invention refers to the use of a genetic construct according to the invention, or of a host cell according to the invention, for identifying whether a gene a protein or a chemical compound is an inhibitor or an activator of the biosynthesis of Q-tRNA. The term "inhibitor" as used herein, refers to a compound which is capable of reducing, in a detectable manner, the biosynthesis of Q-tRNA. The biosynthesis of Q-tRNA is considered reduced when detectable Q-tRNA levels decrease with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by at least 100% (i.e., absent). The term "activator" as used herein, refers to a compound which is capable of increasing, in a detectable manner, the biosynthesis of Q-tRNA. The biosynthesis of Q-tRNA is considered increased when detectable Q-tRNA levels increase with respect to the reference value by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, or more. The reference value refers to the level of detectable Q-tRNA levels in a control sample or in a control experiment.

In another aspect, the invention refers to a method for measuring the concentration of queuosine and/or a queuosine precursor in a sample, preferably in a biological sample, wherein the method comprises:

a) incubating calibration standard dilutions of queuosine and/or queuosine precursor in the presence of a host cell according to the invention in a cell culture medium;
b) incubating a sample dilution in the presence of a host cell according to the invention in a cell culture medium;
c) quantifying the signal associated with the reporter protein in the host cell for the calibration standard dilutions and calculating a calibration curve;
d) quantifying the signal associated with the reporter protein in the host cell for the sample dilution and determining the concentration of queuosine and/or of the queuosine precursor in the sample in relation to the calibration curve of step (c).

The host cells according to the invention may be grown in any suitable cell culture medium, which is appropriate for the correct growth of the host cell (that is, media that contains the essential elements for the host organism), and which should be readily apparent to a person skilled in the art. Suitable cell culture mediums in the context of the instant invention are, without limitation, M63 medium, Luria-Bertani (LB), Tryptone Broth, TY broth, Terrific broth, M9 medium, Msgg medium (for *Bacillus subtilis* and others) or MM medium (for *Streptomyces* sp. and others), and the like. Ideally, minimal media are preferable, which do not contain any component that may contain queuosine or any of its precursors.

In another aspect, the invention refers to a method for identifying whether a chemical compound is an inhibitor or an activator of the biosynthesis of Q-tRNA, wherein the method comprises:

a) incubating calibration standard dilutions of queuosine and/or queuosine precursor in the presence of a host cell according to the invention in a cell culture medium, wherein the cell culture medium is a blank control medium;

b) incubating calibration standard dilutions of queuosine and/or queuosine precursor in the presence of a host cell according to the invention in a cell culture medium, wherein the cell culture medium comprises a library of chemical compounds;

c) quantifying the signal associated with the reporter protein in the host cell for the calibration standard dilutions in blank control medium and calculating a first calibration curve;

d) quantifying the signal associated with the reporter protein in the host cell for the library of chemical compounds and calculating a second calibration curve;

e) comparing the first calibration curve obtained in step (c) with the second calibration curve obtained in step (d), wherein significant variation between the first and second calibration curves is indicative that the chemical compound is an inhibitor or an activator of the biosynthesis of Q-tRNA.

In another aspect, the invention refers to a method for identifying whether a gene or a gene product is an inhibitor or an activator of the biosynthesis of Q-tRNA, wherein the method comprises:

a) incubating calibration standard dilutions of queuosine and/or queuosine precursor in the presence of a host cell according to the invention in a cell culture medium;

b) incubating calibration standard dilutions of queuosine and/or queuosine precursor in the presence of a host cell according to the invention in a cell culture medium, wherein the host cell has been transfected with a metagenomic library;

c) quantifying the signal associated with the reporter protein in the host cell for the calibration standard dilutions in blank control medium and calculating a first calibration curve;

d) quantifying the signal associated with the reporter protein in the host cell for the library of chemical compounds and calculating a second calibration curve;

e) comparing the first calibration curve obtained in step (c) with the second calibration curve obtained in step (d), wherein significant variation between the first and second calibration curves is indicative that the chemical compound is an inhibitor or an activator of the biosynthesis of Q-tRNA.

In the context of the instant invention, the expression "significant variation" refers to any statistically significant variation (i.e., values are significantly increased, significantly decreased, or significantly not altered after an appropriate statistical analysis is carried out). In a particular embodiment, any statistically significant variation will depend on the standard deviation (S.D.) of the intersect with the "y"-axis and the slope of the calibration curves: if said parameters of the calibration curves of the test sample are statistically different than those of the control sample after repeating several iterations of the experiment, it would be considered that there is a significant difference, and that the chemical compound, gene or gene product could be an inhibitor or activator of the biosynthesis of Q-tRNA. In particular embodiments, 3, 5, 10, 15, 20, 30, 40, 50, 100, or more iterations are needed, in order to determine statistical significance. As the skilled person would readily understand, three (3) iterations can be typically sufficient to obtain statistically significant data. However, if the differences between conditions are small, a greater number of iterations (5 - 10, or even more) may be necessary. The values of the "y"-axis intersect and the slope of the calibration curves are used to calculate enzymatic parameters $V_{max(app)}$ and $K_{M(app)}$, that is, apparent maximum velocity and apparent Michaelis constant for the complete reaction of biosynthesis of Q-tRNA from $preQ_1$ precursor, assuming that all enzymes involved in this process stablish an ordered sequential reaction following Michaelis-Menten kinetics. Thus, in particular embodiments of the invention, the experiment is repeated at least 3 times and the values of the mean and associated standard deviation (SD) for the y-axis intersect and slope parameters are calculated. Next, the unpaired t test (or ANOVA if more than two conditions are compared, or any other appropriate statistical analysis) is applied to study whether the parameters are significantly different between conditions (p-value < 0.05). Other non-parametric methods (without assuming normality) such as the Mann-Whitney method, or methods that do not assume equality of variance, such as the unpaired t test with Welch's correction, can also be used.

Accordingly, the uses and methods of the invention can be used to determine whether a chemical compound, gene or gene product is an inhibitor of activator of the biosynthesis of Q-tRNA, wherein:

- A <u>competitive inhibitor</u> increases $K_{M(app)}$, while $V_{max(app)}$ is not altered.
- A <u>non-competitive inhibitor</u> decreases $V_{max(app)}$, while $K_{M(app)}$ is not altered.
- A <u>mixed inhibitor</u> decreases $V_{max(app)}$ and increases or decreases $K_{M(app)}$.
- An activator increases $V_{max(app)}$ and/or decreases $K_{M(app)}$.

In another aspect, the invention refers to a kit or assay device comprising reagents adequate for detecting and/or measuring queuosine and/or a queuosine precursor in a sample, wherein the kit or assay device comprises a genetic construct according to the invention, or of a host cell according to the invention, and wherein said reagents comprise at least 10% of the reagents present in the kit. In the context of the present invention, "kit" or "assay device" is understood as a product or device containing the different reagents necessary for carrying out the methods of the invention packed in a manner that allows for their transport and storage. Materials suitable for packing the components of the kit include glass, plastic, paper, and the like. The kits of the invention can contain instructions for the appropriate use of the components within the kit. Said instructions can be in the form of printed material or in the form of an electronic support capable of storing instructions such that they can be read by a subject, such as electronic storage media, internet repositories and the like. In a preferred embodiment, the reagents adequate for detecting and/or measuring queuosine and/or a queuosine precursor in a sample comprise at least 10%, at least 20%, at least 30%, at least 40%, at least 50%, at least 60%, at least 70%, at least 80%, at least 90%, at least 95%, at least 99%, or 100% of the total amount of reagents forming the kit.

All the terms and embodiments described herein are equally applicable to all aspects of the invention. It should be noted that, as used in the specification and in the appended claims, the singular forms "a", "an", and "the" include their plural referents unless the context clearly indicates otherwise. Similarly, the term "comprises" or "comprising" as used herein also describes "consists of" or "consisting of" in accordance with generally accepted patent practice.

## EXAMPLES

The following invention is hereby described by way of the following examples, which are to be construed as merely illustrative and not limitative of the scope of the invention.

## Example 1: Queuosine reporter gene construction

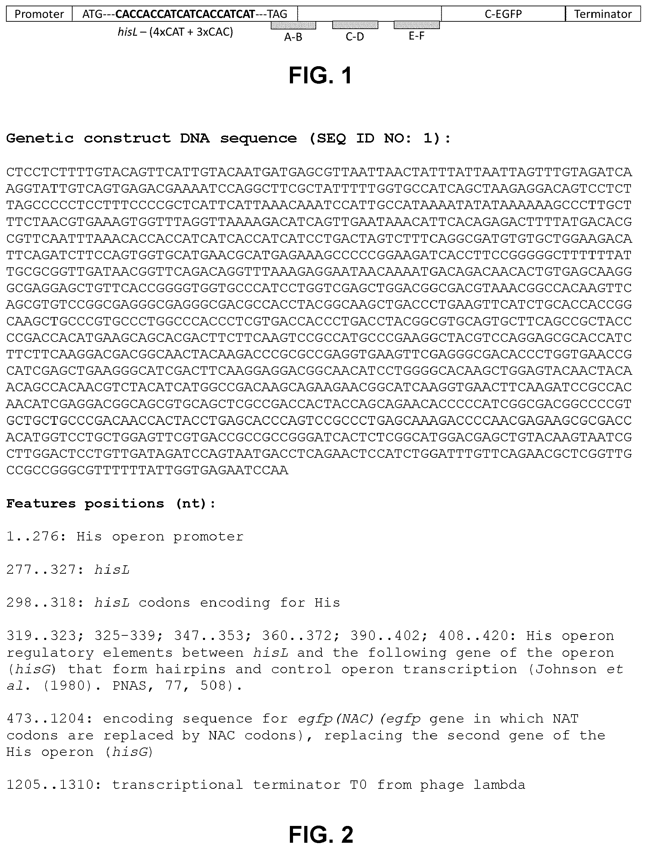

While carrying out research on the biological function of queuosine in bacteria, the inventors observed an overexpression of the histidine (His) operon in a bacterial strain uncapable of producing queuosine *de novo.* They concluded that the regulatory region of the His operon is sensitive to queuosine levels within the cell. In view of these results, the inventors decided to fuse the histidine operon to a reporter gene, where the construct takes advantage of the regulation mechanism of the His operon. This includes a promoter ($P_{his}$) and a leader gene, *hisL*. In particular, the inventors designed a DNA construction in which $P_{his}$, together with *hisL,* were followed by a reporter gene encoding for the Enhanced Green Fluorescence Protein (EGFP), such that the levels of EGFP fluorescence could be correlated with the levels of Q-tRNA(His). The reporter gene replaces the second gene of the His operon, *hisG.* The gene encoding for EGFP was modified to replace all the NAU codons with NAC codons [EGFP(NAC)], so that EGFP translation was not dependent on Q-tRNA levels. Therefore, the invention consists of a construction that comprises the His operon regulatory sequence ($P_{his}$ + *hisL*) followed by a gene encoding for a reporter protein (EGFP) in which all NAU codons are replaced by NAC codons (Figure 1, Figure 2, and SEQ ID NO: 1).

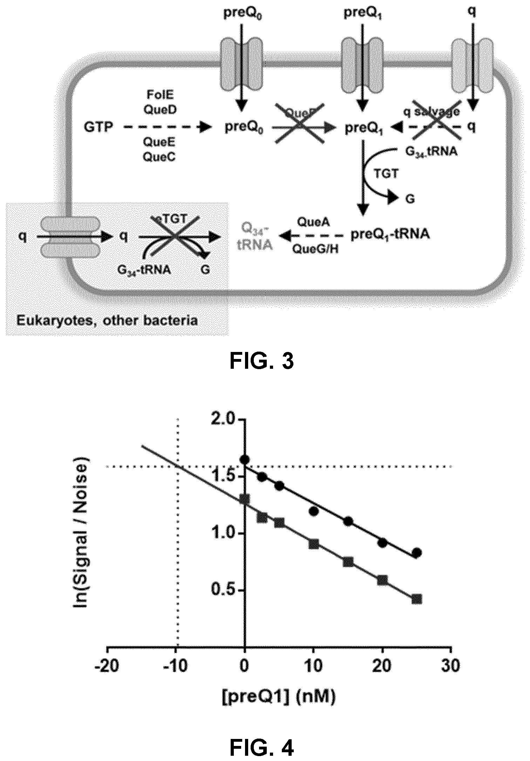

The inventors also introduced this synthetic construct in a cell for measuring Q-tRNA levels. *Escherichia coli* DH10B was used for these experiments. The host bacterium was modified such that Q can only be produced from a certain precursor. Seven enzymes are involved in the biosynthesis of Q in bacteria: QueD, QueE and QueC form the precursor $preQ_0$, which is then converted into the precursor $preQ_1$ by QueF. Then, Tgt catalyzes the $preQ_1$ replacing of the guanine located at position 34 in the tRNAs of His, Asp, Asn and Tyr (NAC/NAU codons) to form $preQ_1$-tRNA. Finally, QueA and QueG transform $preQ_1$-tRNA into Q-tRNA. In addition, *E. coli* can import $preQ_0$ and $preQ_1$ using YhhQ transporter. Other bacteria and eukaryotes can also import queuine, the Q nucleobase, to form Q (Figure 3). The objective of the invention was also to develop a reporter strain for $preQ_1$. For this reason, it was necessary to ensure that all of the Q-tRNA was formed from the exogenous $preQ_1$ of a certain sample, in order to measure $preQ_1$ concentration is said sample. For that purpose, *queF* gene was deleted, preventing the biosynthesis *de novo* of $preQ_1$. Thus, the reporter strain is *E. coli* DH10B *ΔqueF* $P_{his}$-*hisL*-Δ*hisG*::*egfp(NAC).*

## Example 2: preQ$_1$ quantification with E. *coli* DH10B *ΔqueF* P$_{his}$-*hisL*-Δ*hisG*::*egfp(NAC).*

The inventors devised a three-day protocol in order to quantify concentration of queuosine precursor $preQ_1$ using the experimental tools described in Example 1 above.

On the first day of this procedure, 5 ml of LB medium were inoculated with the reporter strain *E. coli* DH10B *ΔqueF*

$P_{his}$-*hisL*-$\Delta$*hisG*::*egfp(NAC)* and incubated at 37 °C and 200 rpm until exponential phase (-8h). Then, 2 ml of the cell culture were centrifuged at 13,000 rpm for 2 min. The supernatant was decanted and discarded, and the cell pellet was resuspended in 1 ml of M63 medium supplemented with Ampicillin (Amp). Finally, the cell pellet was diluted 1/100 in 5 ml of M63-Amp medium and incubated at 37 °C 200 rpm overnight.

The following day, samples and preQ$_1$ standards were prepared as follows:

- preQ$_1$ standard preparation: To prepare preQ$_1$ standards, a 25-$\mu$l aliquot of 10 mM preQ$_1$ is diluted to 25 $\mu$M with M63 medium. Then, a serial dilution was performed to prepare the following preQ$_1$ standard concentration ($\mu$M) in M63 medium: 10, 5, 2.5, 1.5, 1.25, 1, 0.75. 0.5. 0.25, 0. preQ$_1$ standards 0 to 1.5, and 25 $\mu$M were used for test, being finally diluted 1/100; that is, 0-15 nM and 250 nM.
- Sample preparation: samples from which the concentration of preQ$_1$ wanted to be measured were prepared using M63 medium for dilution. The dilution factor of the sample should be within the optimal measure range (5-15 nM). The dilution factor may be optimised using the "Dilution factor test", described in the following paragraph. Samples may need to be pre-treated, as per the "Pre-treatments" section described in the following paragraph.
- Dilution factor test: In order to determine the order of magnitude of preQ$_1$ concentration in the sample of interest, a previous test is needed to set the optimal dilution factor for the sample: (1) Label three 2-mL tubes per each dilution of the sample of interest; (2) Prepare M63 with 2-fold Amp and reporter cells at an OD600 of 0.04. Add 0.3 mL in each tube; (3) Prepare 0.9 mL of 2-fold serial dilutions of the sample, as many dilutions as necessary; (4) Add 0.3 mL of each sample dilution to their corresponding three tubes, notice that sample dilution will be multiplied by two in this step; (5) Prepare standards as described above; (6) Perform the normal protocol with M63 only as a blank experiment; (7) Add 6 $\mu$L of standards 25 $\mu$M, 1$\mu$M, or 0 $\mu$M to the three tubes of each dilution sample; (8) Incubate tubes inside the racks at 37 °C 200 rpm overnight.
- Pre-treatments: In some case, it will be necessary to pre-treat the sample, for instance, because the sample is not sterile, or because reporter strain could not grow in the sample of interest unless some component is removed. There are some strategies that can be used: (1) If the sample is not sterile, it may be filtered. Do not use nitrocellulose filters if the sample has DMSO (for example, for adding a known concentration of preQ$_1$ from standards to check something), because DMSO reacts with the material of some filters, altering the measurement. (2) Treatment with heat: incubation at 100 °C during 30 min does not affect preQ$_1$ concentration. If, for some reason, DMSO has been added to the sample before heating, do not heat the sample in a 15-mL of 50-mL tube, heat it in 1.5-, 2-mL tubes. Some plastics can react with DMSO and measurement is affected. (3) Components of a complex sample can be separated by size using a Microcon centrifugal filter, or similar. (4) It is possible to perform standard protein precipitation with trichloroacetic acid (TCA) or hydrochloric acid (HCl), preQ$_1$ is not affected. Adjust pH and salinity before inoculating the reporter strain in the sample. (5) When measuring preQ$_1$ in biological samples, such as bacterial cultures, serum, urine, etc., it is better to pre-treat the sample with some of the pre-treatments described above for a better growth of the reporter strain. Usually, using 3 KDa Microcon is a very good choice.

On this same day, the cell suspension and cell cultures are prepared as follows:

- Cell suspension: 5-ml culture inoculated overnight was centrifuged at 6,000 rpm 5 min. The supernatant was decanted and discarded, and the cell pellet was resuspended in 1 ml of M63 medium supplemented with Amp, and its optical density at 600 nm (OD600) was measured. OD600 should be around 6-7. The amount of cell suspension needed to reach a final OD600 of 0.02 when added to 16-ml diluted samples was calculated.
- Cell cultures: 16 ml of a diluted sample (as per sample preparation, above) were mixed with 16 $\mu$l of Amp and the volume of cell suspension such that the OD600 was 0.02. Then, 1.8 ml of said mix were added to one of the tubes of each triplicate together with 18 $\mu$l of each preQ$_1$ standard, and the final volume was split into the three tubes of each triplicate (0.6 ml per tube). The tubes were incubated at 37 °C 200 rpm overnight.

On the final day, a 96-well plate was filled with 200 $\mu$l of the content of each tube. Duplicates for each tube were done. OD600 and fluorescence intensity (excitation: $\lambda$=470 nm, emission: $\lambda$=530 nm) were measured. Optimal gain should be set, if possible. If all fluorescence values are similar and high, probably the sample contains too much preQ$_1$ (>30 nM) and further dilutions are needed.

From these data, the concentration of preQ$_1$ was calculated as follows: (1) The OD600 noise was subtracted from OD600 measurements. (2) Fluorescence intensity was divided by corrected OD600. (3) Each technical duplicate was averaged. (4) The technical averages were divided by the average of the three tubes of 250 nM preQ$_1$ standard (Signal to noise; S/N). At 250 nM, all tRNAs will be Q-modified, so fluorescence must be minimal (noise). In some cases, a higher concentration of preQ$_1$ might be required to reach minimal S/N; for example, with the presence of an inhibitor. (5) S/N values were transformed with natural logarithm (Ln). (6) The relationship between the preQ$_1$ standards concentrations and triplicates of ln (S/N) was modelled by linear regression, fitting a linear equation to said data (see Figure 4). Values of S/N lower than 0.45-0.5 were not used and outliers were removed. (7) The equation of the line, the slope, and the interception with y axis for control and sample were calculated. With software like GraphPad Prism, the standard deviation (S.D.) of y-axis interception value may be calculated.

In order to calculate the concentration of queuosine precursor preQ$_1$, the sample tendency line (linear fit to the scatter plot with circular markers, Figure 4) is extrapolated to calculate the preQ$_1$ concentration necessary to reach the y-intercept of the control tendency line (linear fit to the scatter plot with squared markers, Figure 4). For that, sample y-intercept is subtracted from control y-intercept. Then, it is divided by the slope of the sample tendency line. The absolute value of the result is preQ$_1$ concentration. [preQ$_1$] value is finally multiplied by dilution factor of the sample. Control data refers to M63 medium samples.

Table 1: Equations for calculating [preQ1] concentration.

| Equation 1 | $y = m_{sample} \cdot [preQ1] + y_{sample\ intercept}$ |
|---|---|
| Equation 2 | $y_{control\ intercept} = m_{sample} \cdot [preQ1] + y_{sample\ intercept}$ |
| Equation 3 | $[preQ1] = \left\lvert \dfrac{y_{control\ intercept} - y_{sample\ intercept}}{m_{sample}} \right\rvert$ |
| Equation 4 | $[preQ1]S.D. = \left\lvert \dfrac{y_{sample\ intercept}\ S.D.}{m_{sample}} \right\rvert$ |

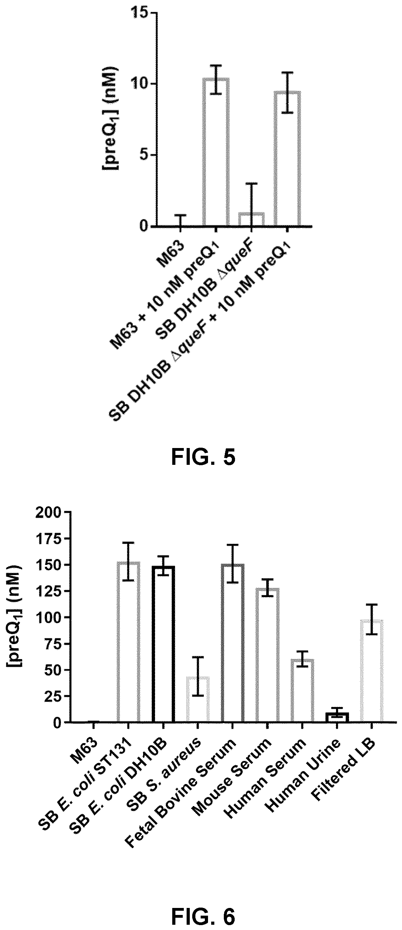

As control experiments, Figure 5 shows the preQ$_1$ concentration obtained for a sample containing M63 medium alone, M63 medium plus 10 nM of preQ$_1$, culture supernatant of an *E. coli* DH10B *queF* mutant strain that does not produce preQ$_1$ (*E. coli* DH10B *ΔqueF*), and the same supernatant plus 10 nM of preQ$_1$. As expected, preQ$_1$ was only detected on the samples to which an exogenous preQ$_1$ has been added.

To prove the suitability of the genetic construct of the invention, preQ$_1$ concentration was measured in supernatants of different *E. coli* strains (ST131, DH10B), in *Staphylococcus aureus* and, in sera from foetal bovine, mouse and human, human urine, and in filtered LB (see Figure 6).

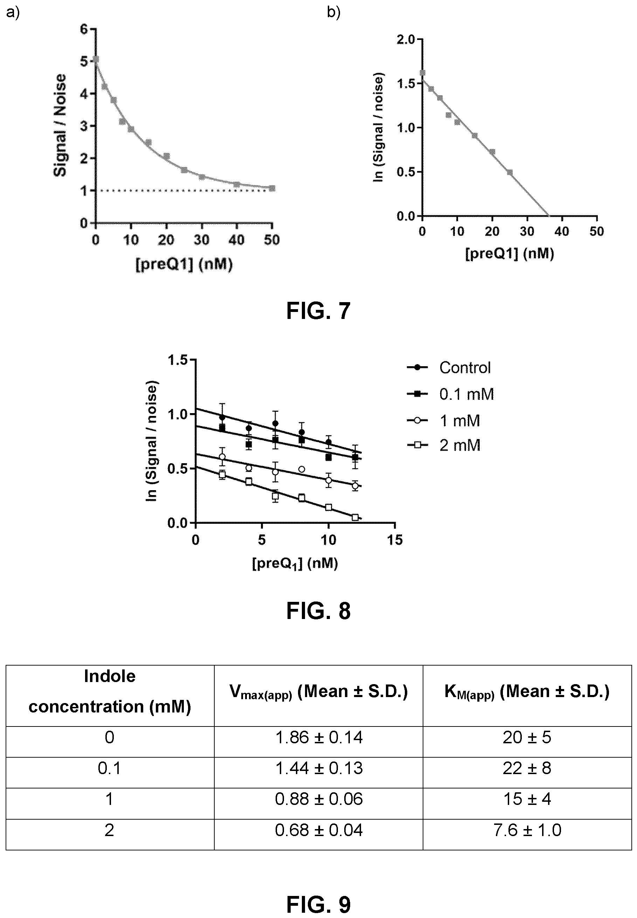

Thus, the EGFP(NAC) fluorescence levels of the reporter strain can be correlated to the levels of preQ$_1$ in a sample where preQ$_1$ concentration needs to be measured. Using the *ΔqueF* mutant from *Escherichia coli*, preQ$_1$ is imported by the YhhQ transporter, increasing Q-tRNA(His) levels, which reduces the translation of the P$_{his}$-*hisL*-Δ*hisG*::*egfp(NAC)* construction, and therefore, the fluorescence levels. The higher the preQ$_1$ concentration levels in the sample, the lower the fluorescence signal (see Figure 7).

**Example 3: Use of queuosine reporter gene construction to search for novel genes, proteins or chemical compounds that inhibit the biosynthesis of Q-tRNA**

The above reporter gene construction can be used to search for novel genes, proteins or compounds that inhibit the biosynthesis of Q-tRNA. For that purpose, two different strategies can be designed:

i) the strain *E. coli* DH10B *ΔqueF* P$_{his}$-*hisL*-Δ*hisG*::*egfp(NAC)* can be incubated with a library of compounds to observe whether the quantitative relationship between the extracellular preQ$_1$ concentration and fluorescence changes in the presence of a specific compound, when compared with the results of incubating the reporter strain in M63 medium (control), and

ii) the strain *E. coli* DH10B *ΔqueF* P$_{his}$-*hisL*-Δ*hisG*::*egfp(NAC)* can be transformed with metagenomic libraries to study whether the expression of certain genes from environmental organisms could alter the relationship between the extracellular preQ$_1$ concentration and fluorescence.

For the first strategy, the effect of indole in the biosynthesis of Q has been tested. The main reason is that indole has been characterised has a quorum sensing molecule that seems to inhibit biofilm formation and virulence in certain bacteria, while Q and/or its precursors appear to work as quorum sensing molecules that produce the opposite effect, that is, they increase biofilm formation and virulence in bacteria. Thus, the strain *E. coli* DH10B *ΔqueF* P$_{his}$-*hisL*-Δ*hisG*:: *egfp(NAC)* was incubated with and without different concentrations of indole, and the linear regressions between the extracellular preQ$_1$ concentration and fluorescence were modelled (see Figure 8). For each indole concentration, slope and "y"-axis intercept are calculated. These parameters would be used to calculate the parameters V$_{max(app)}$ and K$_{M(app)}$,

that is, apparent maximum velocity and apparent Michaelis constant for the complete reaction of biosynthesis of Q-tRNA from preQ$_1$ precursor, assuming that all enzymes involved in this process stablish an ordered sequential reaction following Michaelis-Menten kinetics.

- Apparent maximum velocity of the global reaction is reached when the added preQ$_1$ substrate is present in excess. The more apparent maximum velocity, the bigger the difference of fluorescence measurement in the presence of 0 nM preQ$_1$ and excess preQ$_1$. Therefore, V$_{max(app)}$ would be proportional to "y"-axis intersect (see Table 2).
- Apparent Michaelis constant of the global reaction is defined as the concentration of substrate needed to reach the half of the maximum velocity (see Table 2).

Table 2: Equations for calculating V$_{max(app)}$ and K$_{M(app)}$.

| Equation 5 | $(1)\ V_{max\,(app)} \propto eyintercept\ \text{-}\ 1$ |
|---|---|
| Equation 6 | $(2)\ K_{M(app)} = -\dfrac{ln\left(\dfrac{e^{y_{intercept}}-1}{2}+1\right)}{Slope}$ |

An indole dose-dependent decrease in V$_{max(app)}$ and K$_{M(app)}$ was observed (see Figure 9). Therefore, this result supports that indole may be inhibiting the modification of tRNA with Q, probably as a mixed inhibitor. This approach could similarly be scaled up to search for Q-tRNA inhibitors in big libraries of compounds.

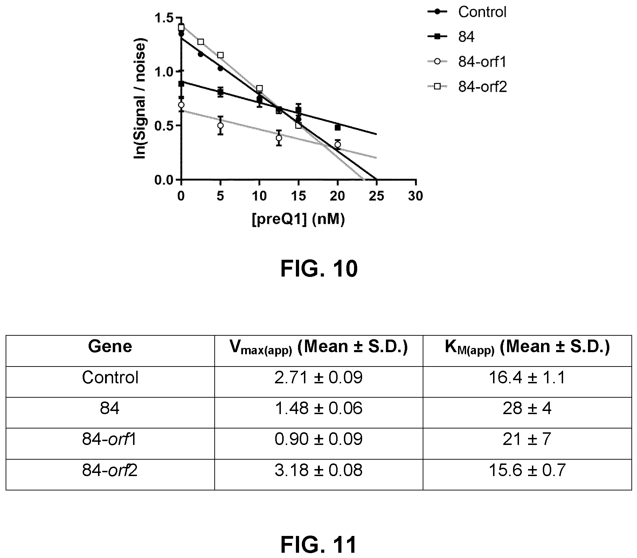

For the second strategy, the search of genes from environmental organisms that inhibit the Q-modification of tRNA was addressed. For that purpose, the strain *E. coli* DH10B *ΔqueF* P$_{his}$-*hisL*-*ΔhisG*::*egfp(NAC)* was transformed with a metagenomic library constructed using environmental DNA isolated from a rhizosphere of an Antarctic plant, harboured into the plasmid pBluescript (pSKII+). Transformation with the plasmid number 84 was observed to produce a decrease in V$_{max(app)}$, but an increase in K$_{M(app)}$ (see Figure 10 and Figure 11). Furthermore, the environmental DNA fragment harboured in plasmid 84 contained two genes: *84-orf1* and 84-*orf2*. It was separately tested which gene produced the observed effect, and data indicated that gene 84-*orf1* was individually responsible for the phenotype (see Figure 10 and Figure 11). Therefore, these results suggest that 84-*orf1* encodes for a gene product that may act as a mixed inhibitor of the Q-modification of tRNAs. In a similar fashion, metagenomic libraries harboured in different plasmids, fosmids or cosmids could be transformed into a strain with the construction of the invention, and it could be used to perform high-throughput screenings (by coupling with microfluidics, for example) to search for genes that inhibit the Q-modification of tRNAs.

**Claims**

1. A genetic construct, wherein the genetic construct comprises:

    - the promoter of the histidine operon (P$_{his}$);
    - the histidine leader gene (*hisL*);
    - the His operon regulatory elements between *hisL* and the following gene of the His operon (*hisG*);
    - a gene encoding for a reporter protein replacing *hisG*, and wherein all NAU codons in the gene encoding for the reporter protein have been modified to NAC codons;
    - a transcriptional terminator.

2. The genetic construct according to claim 1, wherein the reporter protein is EGFP(NAC).

3. The genetic construct according to claim 2, wherein the construct has a sequence with an 80% homology to SEQ ID NO:1.

4. A host cell, wherein the host cell can only use exogenous queuosine or an exogenous queuosine precursor selected from the group consisting of preQ$_0$, preQ$_1$, and q, and wherein the host cell comprises a genetic construct according to any one of claims 1 to 3.

5. The host cell according to claim 4, wherein the host cell is a *ΔqueF* mutant.

6.  The host cell according to claim 5, wherein the host cell is a *ΔqueF* mutant strain of *Escherichia coli* or a *ΔqueF* mutant strain of *Bacillus subtilis*.

7.  Use of a genetic construct according to any one of claims 1 to 3, or of a host cell according to any one of claims 4 to 6, for measuring the degree of queuosine modification of tRNA in a cell, for detecting queuosine and/or a queuosine precursor in a sample, preferably in a biological sample, or for measuring queuosine and/or a queuosine precursor in a sample, preferably in a biological sample.

8.  Use of a genetic construct according to any one of claims 1 to 3, or of a host cell according to any one of claims 4 to 6, for identifying whether a gene, a protein or a chemical compound is an inhibitor or an activator of the biosynthesis of Q-tRNA.

9.  A method for measuring the concentration of queuosine and/or a queuosine precursor in a sample, preferably in a biological sample, wherein the method comprises:

    a) incubating calibration standard dilutions of queuosine and/or queuosine precursor in the presence of a host cell according to any one of claims 4 to 6 in a cell culture medium;
    b) incubating a sample dilution in the presence of a host cell according to any one of claims 4 to 6 in a cell culture medium;
    c) quantifying the signal associated with the reporter protein in the host cell for the calibration standard dilutions and calculating a calibration curve;
    d) quantifying the signal associated with the reporter protein in the host cell for the sample dilution and determining the concentration of queuosine and/or of the queuosine precursor in the sample in relation to the calibration curve of step (c).

10. A method for identifying whether a chemical compound is an inhibitor or an activator of the biosynthesis of Q-tRNA, wherein the method comprises:

    a) incubating calibration standard dilutions of queuosine and/or queuosine precursor in the presence of a host cell according to any one of claims 4 to 6 in a cell culture medium, wherein the cell culture medium is a blank control medium;
    b) incubating calibration standard dilutions of queuosine and/or queuosine precursor in the presence of a host cell according to any one of claims 4 to 6 in a cell culture medium, wherein the cell culture medium comprises a library of chemical compounds;
    c) quantifying the signal associated with the reporter protein in the host cell for the calibration standard dilutions in blank control medium and calculating a first calibration curve;
    d) quantifying the signal associated with the reporter protein in the host cell for the library of chemical compounds and calculating a second calibration curve;
    e) comparing the first calibration curve obtained in step (c) with the second calibration curve obtained in step (d), wherein significant variation between the first and second calibration curves is indicative that the chemical compound is an inhibitor or an activator of the biosynthesis of Q-tRNA.

11. A method for identifying whether a gene or a gene product is an inhibitor or an activator of the biosynthesis of Q-tRNA, wherein the method comprises:

    a) incubating calibration standard dilutions of queuosine and/or queuosine precursor in the presence of a host cell according to any one of claims 4 to 6 in a cell culture medium;
    b) incubating calibration standard dilutions of queuosine and/or queuosine precursor in the presence of a host cell according to any one of claims 4 to 6 in a cell culture medium, wherein the host cell has been transformed or transfected with a metagenomic library;
    c) quantifying the signal associated with the reporter protein in the host cell for the calibration standard dilutions in blank control medium and calculating a first calibration curve;
    d) quantifying the signal associated with the reporter protein in the host cell for the library of chemical compounds and calculating a second calibration curve;
    e) comparing the first calibration curve obtained in step (c) with the second calibration curve obtained in step (d), wherein significant variation between the first and second calibration curves is indicative that the chemical compound is an inhibitor or an activator of the biosynthesis of Q-tRNA.

**12.** A kit or assay device comprising reagents adequate for detecting and/or measuring queuosine and/or a queuosine precursor in a sample, wherein the kit comprises a genetic construct according to any one of claims 1 to 3, or of a host cell according to any one of claims 4 to 6, and wherein said reagents comprise at least 10% of the reagents present in the kit.

| Promoter | ATG---**CACCACCATCATCACCATCAT**---TAG | | | | C-EGFP | Terminator |

*hisL* – (4xCAT + 3xCAC)     A-B    C-D    E-F

# FIG. 1

**Genetic construct DNA sequence (SEQ ID NO: 1):**

```
CTCCTCTTTTGTACAGTTCATTGTACAATGATGAGCGTTAATTAACTATTTATTAATTAGTTTGTAGATCA
AGGTATTGTCAGTGAGACGAAAATCCAGGCTTCGCTATTTTTGGTGCCATCAGCTAAGAGGACAGTCCTCT
TAGCCCCCTCCTTTCCCCGCTCATTCATTAAACAAATCCATTGCCATAAAATATATAAAAAAGCCCTTGCT
TTCTAACGTGAAAGTGGTTTAGGTTAAAAGACATCAGTTGAATAAACATTCACAGAGACTTTTATGACACG
CGTTCAATTTAAACACCACCATCATCACCATCATCCTGACTAGTCTTTCAGGCGATGTGTGCTGGAAGACA
TTCAGATCTTCCAGTGGTGCATGAACGCATGAGAAAGCCCCCGGAAGATCACCTTCCGGGGGCTTTTTTAT
TGCGCGGTTGATAACGGTTCAGACAGGTTTAAAGAGGAATAACAAAATGACAGACAACACTGTGAGCAAGG
GCGAGGAGCTGTTCACCGGGGTGGTGCCCATCCTGGTCGAGCTGGACGGCGACGTAAACGGCCACAAGTTC
AGCGTGTCCGGCGAGGGCGAGGGCGACGCCACCTACGGCAAGCTGACCCTGAAGTTCATCTGCACCACCGG
CAAGCTGCCCGTGCCCTGGCCCACCCTCGTGACCACCCTGACCTACGGCGTGCAGTGCTTCAGCCGCTACC
CCGACCACATGAAGCAGCACGACTTCTTCAAGTCCGCCATGCCCGAAGGCTACGTCCAGGAGCGCACCATC
TTCTTCAAGGACGACGGCAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGGCACAAGCTGGAGTACAACTACA
ACAGCCACAACGTCTACATCATGGCCGACAAGCAGAAGAACGGCATCAAGGTGAACTTCAAGATCCGCCAC
AACATCGAGGACGGCAGCGTGCAGCTCGCCGACCACTACCAGCAGAACACCCCCATCGGCGACGGCCCCGT
GCTGCTGCCCGACAACCACTACCTGAGCACCCAGTCCGCCCTGAGCAAAGACCCCAACGAGAAGCGCGACC
ACATGGTCCTGCTGGAGTTCGTGACCGCCGCCGGGATCACTCTCGGCATGGACGAGCTGTACAAGTAATCG
CTTGGACTCCTGTTGATAGATCCAGTAATGACCTCAGAACTCCATCTGGATTTGTTCAGAACGCTCGGTTG
CCGCCGGGCGTTTTTTATTGGTGAGAATCCAA
```

**Features positions (nt):**

1..276: His operon promoter

277..327: *hisL*

298..318: *hisL* codons encoding for His

319..323; 325-339; 347..353; 360..372; 390..402; 408..420: His operon
regulatory elements between *hisL* and the following gene of the operon
(*hisG*) that form hairpins and control operon transcription (Johnson *et
al.* (1980). PNAS, 77, 508).

473..1204: encoding sequence for *egfp(NAC)* (*egfp* gene in which NAT
codons are replaced by NAC codons), replacing the second gene of the
His operon (*hisG*)

1205..1310: transcriptional terminator T0 from phage lambda

# FIG. 2

FIG. 3

FIG. 4

FIG. 5

FIG. 6

a)

b)

FIG. 7

FIG. 8

| Indole concentration (mM) | $V_{max(app)}$ (Mean ± S.D.) | $K_{M(app)}$ (Mean ± S.D.) |
|---|---|---|
| 0 | 1.86 ± 0.14 | 20 ± 5 |
| 0.1 | 1.44 ± 0.13 | 22 ± 8 |
| 1 | 0.88 ± 0.06 | 15 ± 4 |
| 2 | 0.68 ± 0.04 | 7.6 ± 1.0 |

FIG. 9

FIG. 10

| Gene | $V_{max(app)}$ (Mean ± S.D.) | $K_{M(app)}$ (Mean ± S.D.) |
|------|------|------|
| Control | 2.71 ± 0.09 | 16.4 ± 1.1 |
| 84 | 1.48 ± 0.06 | 28 ± 4 |
| 84-*orf*1 | 0.90 ± 0.09 | 21 ± 7 |
| 84-*orf*2 | 3.18 ± 0.08 | 15.6 ± 0.7 |

FIG. 11

Europäisches
Patentamt

European
Patent Office

Office européen
des brevets

**EUROPEAN SEARCH REPORT**

**Application Number**

EP 24 38 2121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROTH ADAM ET AL: "A riboswitch selective for the queuosine precursor preQ1 contains an unusually small aptamer domain", NATURE STRUCTURAL & MOLECULAR BIOLOGY, NATURE PUBLISHING GROUP US, NEW YORK, vol. 14, no. 4, 1 April 2007 (2007-04-01), pages 308-317, XP002567683, ISSN: 1545-9993, DOI: 10.1038/NSMB1224 [retrieved on 2007-03-25] * the whole document * | 1-12 | INV. C12N15/63 C07K14/47 C12N15/70 |
| X | CHUMLEY FORREST G ET AL: "Genetic Fusions That Place the Lactose Genes Under Histidine Operon Control", J. MOL. BIOL., vol. 145, no. 4, 5 February 1981 (1981-02-05), pages 697-712, XP093186900, | 1 | |
| A | * the whole document * | 2-12 | |
| A | ROBERT K. KULIS-HORN ET AL: "Histidine biosynthesis, its regulation and biotechnological application in Corynebacterium glutamicum", MICROBIAL BIOTECHNOLOGY, vol. 7, no. 1, 25 April 2013 (2013-04-25), pages 5-25, XP055367989, GB ISSN: 1751-7915, DOI: 10.1111/1751-7915.12055 * figures 2-4 * * the whole document * | 1-12 | **TECHNICAL FIELDS SEARCHED (IPC)** C12N C07K |

-/--

The present search report has been drawn up for all claims

| Place of search | Date of completion of the search | Examiner |
|---|---|---|
| Munich | 19 July 2024 | Weinberg, Suzanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 24 38 2121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JUNG SAMIL ET AL: "Transcriptional regulation of histidine biosynthesis genes in Corynebacterium glutamicum", CANADIAN JOURNAL OF MICROBIOLOGY, vol. 56, no. 2, 1 February 2010 (2010-02-01), XP093187209, DOI: doi/10.1139/W09-115 * figure 2 * * the whole document * | 1-12 | |
| A | DÍAZ-RULLO JORGE ET AL: "tRNA queuosine modification is involved in biofilm formation and virulence in bacteria", NUCLEIC ACIDS RESEARCH, vol. 51, no. 18, 28 August 2023 (2023-08-28), pages 9821-9837, XP093187135, GB ISSN: 0305-1048, DOI: 10.1093/nar/gkad667 Retrieved from the Internet: URL:https://academic.oup.com/nar/article-pdf/51/18/9821/51975112/gkad667.pdf> * the whole document * | 1-12 | |

TECHNICAL FIELDS
SEARCHED       (IPC)

The present search report has been drawn up for all claims

| Place of search | Date of completion of the search | Examiner |
|---|---|---|
| Munich | 19 July 2024 | Weinberg, Suzanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JOHNSON et al.** Model for regulation of the histidine operon of Salmonella.. *Proc. Natl. Acad. Sci. USA*, 1980, vol. 77, 508-512